# EUROPÄISCHE PATENTSCHRIFT

(11) **EP 1 556 141 B1**
(45) Veröffentlichungstag und Bekanntmachung des Hinweises auf die Patenterteilung: **29.10.2008**
(21) Anmeldenummer: 03761419.5
(22) Anmeldetag: 16.06.2003
(51) Int. Cl.: A61P 31/10, A61K 38/47

(54) **VERFAHREN ZUR PROPHYLAXE UND THERAPIE VON MYKOSEN BEI FISCHEN UND WIRBELLOSEN UND DEREN ENTWICKLUNGSSTADIEN**
METHOD FOR THE PROPHYLAXIS AND THERAPY OF MYCOSES IN FISH AND INVERTEBRATES AND THE DEVELOPMENT STAGES THEREOF
PROCEDE DE PROPHYLAXIE ET DE THERAPIE DE MYCOSES CHEZ DES POISSONS ET INVERTEBRES ET LEURS STADES DE DEVELOPPEMENT

(30) Priorität: 26.06.2002 DE 10228627
(43) Veröffentlichungstag der Anmeldung: 27.07.2005
(73) Patentinhaber: Biopract GmbH, 12489 Berlin (DE)
(72) Erfinder: RINGPFEIL, Manfred, 97647 Sondheim v.d. Rhön (DE); GERHARDT, Matthias, 12527 Berlin (DE); WOLF, Monika, 12559 Berlin (DE); BLOKESCH, Axel, 13437 Berlin (DE); STEINBERG, Christian, 15566 Schöneiche (DE); KNOPF, Klaus, 12555 Berlin (DE); MEINELT, Thomas, 12689 Berlin (DE); KLOAS, Werner, 12589 Berlin (DE)
(74) Vertreter: Baumbach, Friedrich
(86) Internationale Anmeldenummer: PCT/DE2003/002020
(87) Internationale Veröffentlichungsnummer: WO 2004/002574

(56) Entgegenhaltungen:
- EP-A- 0 474 347
- US-A1- 2002 048 573
- POPE, A.M.S. AND DAVIES, D.A.L.: "The influence of carbohydrases on the growth of fungal pathogens in vitro and in vivo." POSTGRADUATE MEDICAL JOURNAL, Bd. 55, Nr. 647, 1979, Seiten 674-676, XP009017398
- HONGO K: "MECHANISM OF AUTONOMIC REGULATION OF INTRACELLULAR CALCIUM MOVEMENT AND CONTRACTION IN MAMMALIAN VENTRICULAR MUSCLE" TOKYO JIKEIKAI MEDICAL JOURNAL, Bd. 106, Nr. 4, 1991, Seiten 687-706, XP009017354 ISSN: 0375-9172
- GAMA F M ET AL: "Exo- and endo-glucanolytic activity of cellulases purified from Trichoderma reesei." BIOTECHNOLOGY TECHNIQUES, Bd. 12, Nr. 9, 1998, Seiten 677-681, XP009017397 ISSN: 0951-208X
- PATENT ABSTRACTS OF JAPAN vol. 015, no. 173 (C-0828), 2. Mai 1991 (1991-05-02) & JP 03 038530 A (AJINOMOTO CO INC), 19. Februar 1991 (1991-02-19)

## Beschreibung

Die Erfindung betrifft die Herstellung eines Mittels zur Prophylaxe und Therapie von Mykosen bei Fischen und Wirbellosen und deren Entwicklungsstadien in der Aquakultur und Aquaristik, Anwendungsgebiete sind die Tierhaltung und die Tierproduktion.

Ausgangspunkt der Erfindung ist die Tatsache, dass durch Mykosen bei der Züchtung und Haltung von Nutz- und Zierfischen sowie von Krebsen und anderen Wirbellosen hohe wirtschaftliche Verluste entstehen *(*Bruno,D.W., Wood, B.P., 1999: Saprolegnia and other Oomycetes*. In:* Woo, P.T.K., Bruno, D.W. (Hrsg): Fish Diseases and Disorder. Vol.3 Viral, Bacterial and fungal infections. CAB International, Wallingford*).* Der bisher meist verwendete Wirkstoff zur Prophylaxe und Therapie von Mykosen ist Malachitgrün, eine kanzerogene, mutagene und teratogene Substanz, die in Deutschland nur zur Behandlung von Fischeiern geduldet wird, aber nicht zur Therapie von Fischen zugelassen ist *(*Meyer,F.P.; Jorgenson,T.A., 1983: Teratological and other effects of malachite green on development of rainbow trout and rabbits. Trans.Am.Fish.Soc. 112, 818-824, *(Bundesinstitut für gesundheitlichen Verbraucherschutz und Veterinärmedizin*, *2002).*

S. Kudo et al. (The Journal of Experimental Zoology 259: 392-398 (1991)) beschreibt beta-1,3-glucanase enthaltende Extrakte mit in vitro-antimykotischen Effekten gegenüber dem Pilz Saprolegniaparasitica, welcher von der Körperoberfläche eines Karpfens mit Mykose isoliert und in Petrischalen kultiviert worden ist.

Der Erfindung liegt die Aufgabe zugrunde, ein Agenz zu finden, das zur Herstellung eines Mittels zur Prophylaxe und Therapie von Mykosen bei Fischen und Wirbellosen und deren Entwicklungsstadien in der Aquakultur und Aquarisitk verwendet werden kann.

Die Erfindung wird gemäß den Patentansprüchen realisiert, sie betrifft den Schutz von Fischen und Wirbellosen und deren Entwicklungsstadien vor Mykosen, hervorgerufen durch Pilze der Gattungen *Saprolegnia*, *Achlya, Aphanomyces* und anderer in Aquakulturen bedeutsamer Arten (nachfolgend pathogene Pilze genannt) durch den Einsatz von β - glucanspaltenden Enzymgemischen (nachfolgend als Enzymgemische bezeichnet) aus Mikroorganismen als besagtes Agenz, vorzugsweise der Gattungen *Trichoderma* und *Bacillus.* Dieser Einsatz führt zu einer Hemmung bzw. Lyse pathogener Pilze.

Erfindungsgemäß werden die Enzymgemische dem Wasser von Aquakulturanlagen oder Aquarien zugesetzt und durch ihre Einwirkung die Wachstumshemmung sowie Lyse pathogenen Pilze bewirkt (Aquakulturanlagen in diesem Sinne sind Einrichtungen, die der Erbrütung und Aufzucht von Fischen oder Wirbellosen in Süß-, Brack- oder Salzwasser dienen).
Die Enzymgemische werden dem Wasser je nach Einsatzfall in Konzentrationen von 0,001 bis 10⁵ IU pro Liter zugesetzt (1 IU = die Menge Enzym, die pro Minute 1 µMol Glucoseäquivalente freisetzt). Die Dosis richtet sich nach der Beschaffenheit (organische Belastung) des Wassers in der Aquakulturanlage. In Klarwasser (unbelastetes Wasser) werden bis 10 IU, in Wasser mit Schwebstoffpartikeln bis 30 IU, in Braunwasser (organisch belastetes Wasser) bis 50 IU, und in Kreislaufsystemen mit hoher organischer Belastung bis 100 IU Enzymgemisch pro Liter eingesetzt. Der Aktivitätsspiegel wird durch kontinuierliche oder diskontinuierliche Zugabe aufrechterhalten was bestehende Mykosen zurückdrängt und das Auftreten von Neuinfektionen verhindert.
Die erfindungsgemäße Wirkung wird dadurch erreicht, dass die β-glucanspaltenden Enzyme im Gemisch vorgelegt werden. Diese Gemische werden aus Kulturüberständen und/oder aus Membran- und Zellwandfraktionen von Mikroorganismen gewonnen. Die Komponenten werden vorzugsweise im wässrigen Milieu im Bereich von pH 5,5 bis 9,0 zur Lyse der Zellwand oben genannter Pilzgattungen eingesetzt. Sie werden bei einer Wassertemperatur von 4 bis 34°C eingesetzt. Eine Steigerung der Wirkung der Enzymgemische gegen Mykosen wird dadurch erreicht, dass in Kombination mit diesen Enzymen andere Agenzien, vorzugweise Polysaccharide, Polykationen oder Proteine eingesetzt werden, die an exponierte Zellwandbereiche der pathogenen Pilze binden und gegebenenfalls deren Fibrillenstrukturen auflockern.
Eine Wirkung gegen Mykosen wird weiter dadurch erzielt, dass die Enzymgemische nicht nur frei im wässrigen Milieu der Aquakulturanlagen eingesetzt werden, sondern auch an die Oberfläche (Schleimhaut) zu schützender Organismen oder deren Eier gebunden werden. Diese Vorgehensweise ist dann vorzuziehen, wenn eine hohe Infektionsgefahr einen höheren Enzymgehalt erforderlich macht oder bei hohem Wasserdurchsatz der Aquakulturanlage ein ständiges Zudosieren von Enzymgemisch nicht möglich ist. Die Bindung wird durch Behandlung der Tiere oder deren Eier durch eine zeitlich begrenzte Vorinkubation bei erhöhten Enzymkonzentrationen erreicht, wobei neben dem Enzymgemisch vorzugsweise Stoffe eingesetzt werden, welche eine Bindung der Enzyme verstärken.

Die Erfindung soll nachfolgend durch Beispiele näher erläutert werden.

### Ausführungsbeispiele

### Beispiel 1:

### Verhütung von Mykosen an Fischeiern und Jungfischen durch kontinuierliche Gabe glykanspaltender Enzymgemische

Befruchtete Fischeier werden bis zum Schlupf der Jungfische in üblichen Erbrütungsanlagen gehalten. Jungfische werden in den üblichen Anzuchtbecken gehalten.
Dem Wasser der Erbrütungs- oder Anzuchtanlage wird ein flüssiges glykanspaltendes Enzymgemisch in einer Dosis von 10 IU β-1,4-*endo*-Glucanase und 0,1 U β-1,3-*endo*-Glucanase pro Liter Wasser zugefügt. Die Enzymlösung wird ständig nachdosiert und durch die Wasserbewegung eingemischt. Die Enzymdosis im wässrigen Milieu wird nach der Aktivitätshöhe der im Gemisch enthaltenen β-1,3 und 1,4-*endo*-Glucanase eingestellt.

### Beispiel 2:

### Therapie von Fischmykosen durch Behandlung mit glykanspaltenden Enzymgemischen

Infizierte Fischeier oder Jungfische (bis zur Setzlingsreife) werden in einem Wasserbad mit einer hohen Dosis von 1.000 IU β-1,4-*endo*-Glucanase pro Liter Wasser, für eine Dauer von maximal 8 Stunden behandelt. Neben dem Enzymgemisch wird dem Bad Natriumchlorid in einer Konzentration von 0,1 M zugesetzt, welches die Bindung der Enzyme an die Oberfläche der Fische bzw. der Fischeier festigt. Anschließend werden die Fische bzw. Fischeier Erbrütungs- bzw. Anzuchtbecken- überführt, welche das frisches Wasser und Enzymgemisch entsprechend Beispiel 1 enthalten. Lokale Mykosen können ebenfalls durch Einpinseln der befallenen Bereiche mit Enzymgemisch behandelt werden. Durch die Nachbehandlung entsprechend Beispiel 1 wird das Pilzwachstum dauerhaft gehemmt, Neuinfektionen werden verhindert.

### Beispiel 3:

### Verhütung und Behandlung von Mykosen in der Fischmast in Klarwasseranlagen

Die Fische werden zur Mast in den üblichen Mastbecken gehalten.
Dem Wasser der Anlage wird ein flüssiges glykanhydrolysierendes Enzymgemisch zugefügt und eine Aktivität von 1 Einheit pro Liter eingestellt. Die Enzymlösung wird entsprechend dem Wasseraustausch zudosiert und durch die Wasserbewegung eingemischt. Die Enzymdosis im wässrigen Milieu wird nach der Aktivitätshöhe der im Gemisch enthaltenen β-1,3-*endo-*Glucanase als Leitaktivität eingestellt. Eine kontinuierliche Abnahme des Enzymspiegels mit der Zeit wird akzeptiert. Bei Austausch des Wassers wird eine neue Dosis Enzym zugegeben. Die Dosis wird bei auftretender Infektionsgefahr erhöht.

### Beispiel 4:

### Verhütung von Mykosen durch Prophylaxe beim Transport von Fischen

Unter Transportbedingungen unterliegen Fische gesteigertem Stress und damit erhöhter Infektionsgefahr. Durch den hohen Tierbesatz und durch mechanische Einwirkung beim Keschern und Umsetzen der Fische besteht zudem Verletzungsgefahr. Zur Verhinderung von Primärinfektionen wird dem Wasser der Transportbehälter Enzymgemisch in hohen Dosen bis 10⁵ IU pro Liter zugesetzt. Auskeimende Zoosporen der pathogenen Pilze werden lysiert und damit eine Besiedlung oberflächlicher Wunden durch die pathogenen Pilze wirksam verhindert.

### Beispiel 5:

### Verhütung und Behandlung von Mykosen bei Larvenstadien der Krebse

Dem Wasser der Anzuchtbecken von Krebslarven (Nauplius-, Zoea- und Mysis- Stadien) bis zur Postlarve werden glykanhydrolysierende Enzymgemische in einer Dosis von 10 IU β-1,4-*endo*-Glucanase pro Liter Wasser zugesetzt. Eine Hemmung der pathogenen Pilze wird bei den Krebsen durch Präparate erreicht, die als Hauptaktivität β-1,4-*endo*-Glucanase aufweisen. Der Enzymspiegel wird durch diskontinuierliche Gaben wiederholt aufgefrischt, bis die Tiere das Postlarvenstadium erreicht haben und in die Becken zur Mast umgesetzt werden.

## Patentansprüche

1. Verwendung von N- und O- glykosylspaltenden mikrobiellen Enzymgemischen, welche aus Kulturüberständen und/oder aus Membran- und Zellwandfraktionen von Mikroorganismen gewonnen werden zur Herstellung eines Mittels zur Prophylaxe und Therapie von Mykosen bei Fischen und Wirbellosen und deren Entwicklungsstadien in der Aquakultur und in der Aquaristik (Süß-, Brack- und Salzwasser), und Zugabe dieses Mittels in das wässrige Milieu, wobei die Dosis an β-glucanspaltendem Enzymgemisch 0,001 IU bis 10⁵ IU pro Liter Wasser beträgt und der Aktivitätsspiegel durch kontinuierliche oder diskontinuierliche Zugabe aufrechterhalten wird.

2. Verwendung nach Anspruch 1, **dadurch gekennzeichnet, dass** die Enzymgemische frei im wässrigen Milieu der Aquakulturanlagen verwendet werden.

3. Verwendung nach Anspruch 1 oder 2, **dadurch gekennzeichnet, dass** die Enzymgemische zur Bindung an die Oberfläche zu schützender Organismen oder deren Eier verwendet werden.

4. Verwendung nach Anspruch 3, **dadurch gekennzeichnet, dass** die Enzymgemische mit Stoffen, die die Bindung der Enzyme verstärken, verwendet werden, wobei Ionen, Proteine oder Polysaccharide zugesetzt werden, die das Mittel an die Oberfläche der zu schützenden Tiere oder deren Entwicklungsstadien binden oder in räumlicher Nähe der Oberfläche halten.

5. Verwendung nach Anspruch 1 bis 4, **dadurch gekennzeichnet, dass** die Enzymgemische Glykanhydrolasen, vorzugsweise β - Glucanhydrolasen in unterschiedlichen Anteilen enthalten.

6. Verwendung nach Anspruch 1 bis 5, **dadurch gekennzeichnet, dass** Enzymgemische aus Mikroorganismen der Gattungen *Trichoderma* und *Bacillus* eingesetzt werden.

7. Verwendung nach Anspruch 1 bis 6, **dadurch gekennzeichnet, dass** die Enzymgemische in einer Dosis von 0,1 bis 100 IU β - Glucanhydrolasen in unterschiedlichen Mischungsverhältnissen der Einzelenzyme pro Liter Wasser in der Aquakulturanlage kontinuierlich oder diskontinuierlich zugesetzt werden.

8. Verwendung nach Anspruch 1 bis 7, **dadurch gekennzeichnet, dass** die Enzymgemische im Süsswasser bei pH 5,5 bis 9,0, vorzugsweise bei pH 6,5 bis pH 8,0 eingesetzt werden.

9. Verwendung nach Anspruch 1 bis 8, **dadurch gekennzeichnet, dass** die Enzymgemische im Brack- und Salzwasser bei pH 7,0 bis 9,0, vorzugsweise bei pH 8,0 bis 9,0 eingesetzt werden.

10. Verwendung nach Anspruch 1 bis 9, **dadurch gekennzeichnet, dass** die Enzymgemische bei Temperaturen von 4 bis 34°C, vorzugsweise 7 bis 32°C eingesetzt werden.

11. Verwendung nach Anspruch 1 bis 10, **dadurch gekennzeichnet, dass** die Enzymgemische in Kombination mit weiteren Agenzien, vorzugsweise Polysacchariden, Polykationen, oder Proteinen eingesetzt werden, die an exponierte Zellwandbereiche der pathogenen Pilze binden.

12. Verwendung nach Anspruch 11, **dadurch gekennzeichnet, dass** als Agenzien Polysaccharide oder Polykationen verwendet werden, vorzugsweise modifiziertes Chitin.

13. Verwendung nach Anspruch 11, **dadurch gekennzeichnet, dass** als Agenzien Proteine verwendet werden, vorzugsweise Fibrillen auflockernde Proteine.

## Claims

1. Use of microbial enzyme mixtures cleaving N- and O glycosyl linkages, obtained from culture supernatants and/or from membrane and cell wall fractions of micro-organisms for the production of a means of prophylaxis and therapy of mycoses in fishes and invertebrates and their development phases in aquaculture and aquaristics (freshwater, brackish water and sea water) and addition of said means to the watery milieu, the dosage of β-glucan separating enzyme mixture amounting to 0.001 IU to 10⁵ IU per litre of water and the activity level being maintained by continuous or discontinuous addition.

2. Use according to Claim 1, **wherein** the enzyme mixtures are used freely in the watery milieu of the aquaristic systems.

3. Use according to Claim 1 or 2, **wherein** the enzyme mixtures are used for binding to the surface of organisms or their eggs to be protected.

4. Use according to Claim 3, **wherein** the enzyme mixtures are used with substances reinforcing the binding of the enzymes, in which context ions, proteins or polysaccharides are added, binding the means to the surface of the animals to be protected or their development stages or keeping them in the vicinity of the surface.

5. Use according to Claim 1 to 4, **wherein** the enzyme mixtures contain glycanohydrolases, preferably β - glucanohydrolases in varying quantities.

6. Use according to Claim 1 to 5, **wherein** enzyme mixtures of micro-organisms of the genera *Trichoderma* and *Bacillus* are used.

7. Use according to Claim 1 to 6, **wherein** the enzyme mixtures are continuously or discontinuously added to the aquaculture system in a dosage of 0.1 to 100 IU β - glucanohydrolases per litre of water in various mixing ratios of the individual enzymes.

8. Use according to Claim 1 to 7, **wherein** the enzyme mixtures are used in fresh water at pH 5.5 to 9.0, preferably at pH 6.5 to pH 8.0.

9. Use according to Claim 1 to 8, **wherein** the enzyme mixtures are used in brackish water and salt water at pH 7.0 to 9.0, preferably at pH 8.0 to 9.0.

10. Use according to Claim 1 to 9, **wherein** the enzyme mixtures are used at temperatures from 4 to 34°C, preferably 7 to 32°C.

11. Use according to Claim 1 to 10, **wherein** the enzyme mixtures are used in combination with other agents, preferably polysaccharides, polycations or proteins, which bind to exposed cell wall areas of the pathogenic fungi.

12. Use according to Claim 11, **wherein** polysaccharides or polycations are used as agents, preferably modified chitin.

13. Use according to Claim 11, **wherein** proteins are used as agents, preferably fibril loosening proteins.

## Revendications

1. Emploi de mélanges d'enzymes microbiens clivant des liaisons N et O glycosidiques lesquels enzymes sont extraits de surnageants de culture et/ou de fractions de membrane et de paroi cellulaire de microorganismes pour la fabrication d'un médicament destiné à la prophylaxie et au traitement de mycoses chez les poissons et les invertébrés et leurs stades de développement en aquaculture et en aquaristique (eau douce, saumâtre et salée) et addition de ce médicament dans le milieu aqueux, la dose de mélange enzymatique dégradant le β-glucane s'élevant à 0,001 IU de 10⁵ IU par litre d'eau et le niveau d'activité étant maintenu par addition continue ou discontinue de ce médicament.

2. Emploi selon la revendication 1, **se caractérisant par le fait que** les mélanges enzymatiques sont utilisés à l'état libre dans le milieu aqueux des dispositifs d'aquaculture.

3. Emploi selon la revendication 1 ou 2 **se caractérisant par le fait que** les mélanges enzymatiques sont employés pour leur adsorption à la surface d'organismes à protéger ou de leurs oeufs.

4. Emploi selon la revendication 3 **se caractérisant par le fait que** les mélanges enzymatiques sont employés avec des substances qui renforcent l'adsorption des enzymes, tout en ajoutant des ions, des protéines ou des polysaccharides qui lient le médicament à la surface des animaux à protéger ou de leurs stades de développement ou maintiennent le médicament à proximité de leur surface.

5. Emploi selon les revendications 1 à 4, **se caractérisant par le fait que** les mélanges enzymatiques contiennent des hydrolases de glucane, de préférence des hydrolases de β-glucane, en différentes proportions.

6. Emploi selon les revendications 1 à 5, **se caractérisant par le fait que** des mélanges enzymatiques issus de microorganismes des genres *Trichoderma* et *Bacillus* sont employés.

7. Emploi selon la revendication 1 à 6, **se caractérisant par le fait que** les mélanges enzymatiques sont ajoutés en continu ou en discontinu au dispositif d'aquaculture dans une dose de 0,1 à 100 IU d'hydrolases de β-glucane en différents rapports de mélange des différentes enzymes par litre d'eau.

8. Emploi selon les revendications 1 à 7, **se caractérisant par le fait que** les mélanges enzymatiques sont employés dans de l'eau douce à un pH de 5,5 à 9,0, de préférence à un pH de 6,5 à un pH de 8,0.

9. Emploi selon les revendications 1 à 8, **se caractérisant par le fait que** les mélanges enzymatiques sont employés dans de l'eau saumâtre et dans de l'eau salée à un pH de 7,0 à 9,0, de préférence à un pH de 8,0 à 9,0.

10. Emploi selon les revendications 1 à 9, **se caractérisant par le fait que** les mélanges enzymatiques sont employés à des températures de 4 à 34 °C, de préférence de 7 à 32 °C.

11. Emploi selon les revendications 1 à 10, **se caractérisant par le fait que** les mélanges enzymatiques sont employés en combinaison avec d'autres agents, de préférence des polysaccharides, des polycations ou des protéines qui se lient à des zones exposées de la paroi cellulaire des champignons pathogènes.

12. Emploi selon la revendication 11, **se caractérisant par le fait que** des polysaccharides ou des polycations, de préférence de la chitine modifiée, sont utilisés comme agents.

13. Emploi selon la revendication 11, **se caractérisant par le fait que** des protéines, de préférence des protéines déstructurant les fibrilles sont utilisés comme agents.
